# FASCICULE DE BREVET EUROPEEN

(11) **EP 2 240 197 B1**
(45) Date de publication et mention de la délivrance du brevet: **15.04.2015**
(21) Numéro de dépôt: 09715010.6
(22) Date de dépôt: 09.01.2009
(51) Int. Cl.: A61K 38/17, A61P 27/06

(54) **UTILISATION D'UNE HOMEOPROTEINE DE LA FAMILLE BICOÏD POUR LA PREVENTION OU LE TRAITEMENT DE LA DEGENERESCENCE DES NEURONES GANGLIONNAIRES RETINIENS**
VERWENDUNG VON HOMEOPROTEIN DER BICOID-FAMILIE ZUR PRÄVENTION ODER BEHANDLUNG VON RETINALER GANGLIONEN-NEURONEN-DEGENERATION
USE OF A HOMEOPROTEIN OF THE BICOID FAMILY FOR PREVENTING OR TREATING RETINAL GANGLIONIC NEURONE DEGENERATION

(30) Priorité: 09.01.2008 FR 0800110
(43) Date de publication de la demande: 20.10.2010
(73) Titulaire: Centre National de la Recherche Scientifique, 75016 Paris (FR); Ecole Normale Supérieure, 75230 Paris Cedex 05 (FR)
(72) Inventeur: PROCHIANTZ, Alain, F-75006 Paris (FR); MOYA, Kenneth, Lee, F-92110 Clichy (FR)
(74) Mandataire: Leblois-Préhaud, Hélène Marthe Georgette
(86) Numéro de dépôt international: PCT/FR2009/000031
(87) Numéro de publication internationale: WO 2009/106767

(56) Documents cités:
- EP-A- 1 591 127
- NISHIDA A ET AL: "Otx2 homeobox gene controls retinal photoreceptor cell fate and pineal gland development" NATURE NEUROSCIENCE, NATURE AMERICA, INC, US, vol. 6, no. 12, 16 novembre 2003 (2003-11-16), pages 1255-1263, XP002979640 ISSN: 1097-6256
- SAKAMI S ET AL: "Expression of Otx2 during regeneration and development of newt regina" ZOOLOGICAL SCIENCE, ZOOLOGICAL SOCIETY OF JAPAN, TOKYO, JP, vol. 18, no. SUPPL, 1 janvier 2001 (2001-01-01), page 64, XP002979637 ISSN: 0289-0003
- RATH ET AL: "Ontogenetic expression of the Otx2 and Crx homeobox genes in the retina of the rat" EXPERIMENTAL EYE RESEARCH, ACADEMIC PRESS LTD., LONDON, vol. 85, no. 1, 1 juillet 2007 (2007-07-01), pages 65-73, XP022153829 ISSN: 0014-4835

### SEQUENCE LISTING

<110> - CENTRE NATIONAL DE LA RECHERCHE SCIENTIFIQUE
   - ECOLE NORMALE SUPERIEURE
   - PROCHIANTZ, Alain
   - MOYA, Kenneth Lee
<120> UTILISATION D'UNE HOMEOPROTEINE DE LA FAMILLE BICOÏD POUR LA PREVENTION OU LE TRAITEMENT DE LA DEGENERESCENCE DES NEURONES GANGLIONNAIRES RETINIENS
<130> - MJP/mad-F644-174/PCT
<150> FR08/00110
   <151> 2008-01-09
<160> 2
<170> PatentIn version 3.3
<210> 1
   <211> 289
   <212> PRT
   <213> Homo sapiens
<220>
   <221> DOMAIN
   <222> (38)..(97)
   <223> Homéodomaine
<400> 1
<210> 2
   <211> 297
   <212> PRT
   <213> Homo sapiens
<220>
   <221> DOMAIN
   <222> (32)..(97)
   <223> Homéodomaine
<220>
   <221> DOMAIN
   <222> (46) .. (105)
   <223> Homéodomaine
<400> 2
   Met Met Ser Tyr Leu Lys Gln Pro Pro Tyr Ala Val Asn Gly Leu Ser
   1               5                   10                  15
   Leu Thr Thr Ser Gly Met Asp Leu Leu His Pro Ser Val Gly Tyr Pro
               20                  25                  30
   Gly Pro Trp Ala Ser Cys Pro Ala Ala Thr Pro Arg Lys Gln Arg Arg
           35                  40                  45
   Glu Arg Thr Thr Phe Thr Arg Ala Gln Leu Asp Val Leu Glu Ala Leu
       50                  55                  60
   Phe Ala Lys Thr Arg Tyr Pro Asp Ile Phe Met Arg Glu Glu Val Ala
   65                  70                  75                  80
   Leu Lys Ile Asn Leu Pro Glu Ser Arg Val Gln Val Trp Phe Lys Asn
                   85                  90                  95
   Arg Arg Ala Lys Cys Arg Gln Gln Gln Gln Gln Gln Gln Asn Gly Gly
               100                 105                 110
   Gln Asn Lys Val Arg Pro Ala Lys Lys Lys Thr Ser Pro Ala Arg Glu
           115                 120                 125
   Val Ser Ser Glu Ser Gly Thr Ser Gly Gln Phe Thr Pro Pro Ser Ser
       130                 135                 140
   Thr Ser Val Pro Thr Ile Ala Ser Ser Ser Ala Pro Val Ser Ile Trp
   145                 150                 155                 160
   Ser Pro Ala Ser Ile Ser Pro Leu Ser Asp Pro Leu Ser Thr Ser Ser
                   165                 170                 175
   Ser Cys Met Gln Arg Ser Tyr Pro Met Thr Tyr Thr Gln Ala Ser Gly
               180                 185                 190
   Tyr Ser Gln Gly Tyr Ala Gly Ser Thr Ser Tyr Phe Gly Gly Met Asp
           195                 200                 205
   Cys Gly Ser Tyr Leu Thr Pro Met His His Gln Leu Pro Gly Pro Gly
       210                 215                 220
   Ala Thr Leu Ser Pro Met Gly Thr Asn Ala Val Thr Ser His Leu Asn
   225                 230                 235                 240
   Gln Ser Pro Ala Ser Leu Ser Thr Gln Gly Tyr Gly Ala Ser Ser Leu
                   245                 250                 255
   Gly Phe Asn Ser Thr Thr Asp Cys Leu Asp Tyr Lys Asp Gln Thr Ala
               260                 265                 270
   Ser Trp Lys Leu Asn Phe Asn Ala Asp Cys Leu Asp Tyr Lys Asp Gln
           275                 280                 285
   Thr Ser Ser Trp Lys Phe Gln Val Leu
       290                 295

## Description

La présente invention est relative au traitement de maladies impliquant une dégénérescence des neurones ganglionnaires rétiniens (RGCs), et notamment du glaucome.

La rétine est le feuillet cellulaire tapissant le fond de l'oeil. Elle contient différents types de neurones dont le rôle est de capter l'énergie lumineuse et de la transformer en signal nerveux, ainsi que des cellules gliales.

Schématiquement, la rétine comprend trois couches principales de neurones : les neurones photorécepteurs (cônes et bâtonnets), les neurones bipolaires, et les neurones ganglionnaires ; d'autres neurones, les neurones amacrines et les neurones horizontaux, jouent un rôle régulateur. Les neurones photorécepteurs réagissent à la lumière, et le signal qu'ils génèrent est transmis par l'intermédiaire des neurones bipolaires, aux neurones ganglionnaires, dont les axones constituent les fibres nerveuses du nerf optique, assurant l'envoi de l'information au cerveau.

La dégénérescence des neurones rétiniens est impliquée dans diverses rétinopathies. Ainsi, la dégénérescence des neurones photorécepteurs est impliquée dans certaines pathologies, telles que la rétinite pigmentaire ou la dégénérescence maculaire. Dans d'autres pathologies, ce sont les neurones ganglionnaires qui sont atteints. Des atteintes des neurones ganglionnaires rétiniens peuvent être observées dans diverses neuropathies optiques, génétiques ou vasculaires, mais aussi plus largement dans le cadre de maladies neurodégénératives (telle que par exemple la maladie d'Alzheimer, la sclérose en plaques ou la maladie de Parkinson).

L'une des pathologies dans lesquelles le rôle prépondérant de l'atteinte des neurones ganglionnaires rétiniens a été démontré est le glaucome. Dans cette pathologie, la dégénérescence de ces neurones et de leurs axones aboutit à une détérioration lente du nerf optique, qui peut conduire à la cécité totale. La cause la plus fréquente du glaucome est une hyperpression intraoculaire. Bien que les mécanismes aboutissant à la destruction des neurones ganglionnaires soient encore mal élucidés, son implication dans la survenue de la pathologie a été montrée (NICKELLS, 2007, Can. J. Ophthalmol. , 42, 278-87). En outre, chez les patients atteints de glaucome, des concentrations excessives en glutamate, un neurotransmetteur normalement présent dans l'humeur vitrée, ont été observées (DREYER et al., Arch Ophthalmol, 114, 299-305, 1996) (MORRISON et al., Prog Retin Eye Res, 24, 217-240, 2005). A ces concentrations, le glutamate a une activité neurotoxique sur les neurones ganglionnaires en culture ou *in vivo* (HAHN et al., Proc Natl Acad Sci U S A, 85, 6556-6560, 1988; LI et al., Invest Ophthalmol Vis Sci, 40, 1004-1008, 1999) (SHEN and SLAUGHTER, J Neurophysiol, 87, 1629-1634, 2002). Le TNF-alpha est également surexprimé dans la rétine et le nerf optique des patients atteints de glaucome (YUAN and NEUFELD, Glia, 32, 42-50, 2000; TEZEL et al., Invest Ophthalmol Vis Sci, 42, 1787-1794, 2001). La toxicité de cette cytokine, associée à la présence de récepteurs sur les neurones ganglionnaires, a été démontrée *in vitro* (FUCHS et al., Invest Ophthalmol Vis Sci, 46, 2983-2991, 2005) et *in vivo* (FONTAINE et al., J Neurosci, 22, RC216, 2002).

Les traitements actuellement disponibles contre le glaucome reposent sur des molécules capables de baisser la pression intraoculaire (WOODWARD and CHEN, Expert Opin Emerg Drugs, 12, 313-327, 2007).

Les homéoprotéines, ou protéines à homéodomaine sont des facteurs de transcription jouant une rôle majeur dans les phénomènes de migration et différenciation cellulaires impliqués dans la morphogénèse de l'organisme. Elles se caractérisent par la présence d'une séquence de 60 acides aminés, l'homéodomaine, qui est un domaine de liaison à l'ADN, possédant une structure particulière (hélice/tour/hélice). Il a été montré que l'homéodomaine isolé de la protéine Antennapedia de Drosophile peut d'une part traverser la membrane de neurones en culture, et d'autre part s'accumuler dans le noyau et promouvoir la croissance des neurites (Demande EP0485578 (JOLIOT et al., Proc Natl Acad Sci U S A, 88, 1864-1868, 1991). Les propriétés de pénétration de l'homéodomaine d'Antennapedia sont conférées par sa troisième hélice, et apparaissent très conservées entre les homéoprotéines ; ses propriétés sur la croissance des neurites apparaissent corrélées à ses propriétés de liaison à l'ADN, au niveau de sites de liaison définis par la séquence consensus ANNNNCATTA (Demande EP0485578 (JOLIOT et al., Proc Natl Acad Sci U S A, 88, 1864-1868, 1991).

Otx2 (Orthodenticle homolog 2) est une homéoprotéine contenant un homéodomaine de type bicoïd (SIMEONE et al., Embo J, 12, 2735-2747, 1993). Elle appartient à la famille d'homéoprotéines Otx, qui joue un rôle fondamental dans le développement du cerveau au cours de l'embryogenèse (ACAMPORA et al., Prog Neurobiol, 64, 69-95, 2001; SIMEONE et al., Curr Opin Genet Dev, 12, 409-415, 2002). Il a également été montré qu'Otx2 intervenait dans la formation de la rétine, en favorisant la différenciation de cellules souches rétiniennes en neurones photorécepteurs. La Demande EP1591127 rapporte ainsi que la transformation de cellules souches rétiniennes par un vecteur recombinant exprimant Otx2, induit la différenciation de ces cellules en neurones photorécepteurs, au détriment des autres types de neurones rétiniens, et propose l'utilisation d'Otx2 pour traiter diverses pathologies rétiniennes impliquant une dégénérescence des neurones photorécepteurs.

Les Inventeurs ont maintenant mis en évidence un nouvel effet d'Otx2, qui ne se manifeste pas au niveau de la différenciation des neurones rétiniens, mais à celui de la survie des neurones adultes déjà différenciés, et qui concerne les neurones ganglionnaires. Ils ont en effet observé que l'addition d'Otx2 à des cultures de neurones ganglionnaires adultes axotomisés (qui habituellement meurent très rapidement), permettait leur survie.

Cette nouvelle propriété d'Otx2 permet de proposer son utilisation pour améliorer la survie des neurones ganglionnaires adultes en culture *in vitro,* ainsi que pour la prévenir ou traiter *in vivo* la dégénérescence des neurones ganglionnaires.

On définit par « homéoprotéine Otx2 » toute homéoprotéine dont l'homéodomaine possède au moins 98% d'identité de séquence avec celui de la protéine Otx2 humaine (résidus 38-97 de la séquence SEQ ID NO: 1), et qui contient un résidu lysine en position 50 dudit homéodomaine, et dont la séquence polypeptidique globale possède au moins 90%, de préférence au moins 95% d'identité avec la protéine SEQ ID NO: 1 (correspondant à l'isoforme 1 de la protéine Otx2 humaine, référencée sur SwissProt sous le numéro P32243), ou avec la protéine SEQ ID NO: 2 (correspondant à l'isoforme 2 de la protéine Otx2 humaine, référencée sur SwissProt sous le numéro P32243-2).

Ladite homéoprotéine Otx2 peut être facilement obtenue par des méthodes bien connues en elles-mêmes. Elle peut par exemple être produite sous forme recombinante par les méthodes classiques de génie génétique.

Plus spécifiquement, la présente invention a pour objet l'utilisation d'une homéoprotéine Otx2, ou d'une composition comprenant ladite homéoprotéine Otx2 comme médicament pour la prévention ou le traitement de la dégénérescence des neurones ganglionnaires, et plus particulièrement d'une telle dégénérescence intervenant au cours du glaucome.

La présente invention peut en particulier être mise en oeuvre chez des patients ne présentant pas de dégénérescence des neurones photorécepteurs.

La présente Invention a également pour objet l'utilisation d'une homéoprotéine Otx2, ou d'une composition comprenant ladite homéoprotéine pour augmenter la survie de neurones ganglionnaires rétiniens en culture.

Pour la mise en oeuvre de la présente invention, il suffit de mettre ladite homéoprotéine en contact avec les neurones ganglionnaires ; elle pénètre en effet dans ceux-ci grâce à la séquence d'internalisation présente dans sa troisième hélice. De manière préférée ladite mise en contact est effectuée à une concentration de ladite homéoprotéine de 0,5 à 10 nM, avantageusement de 1 à 5 nM, et de manière particulièrement avantageuse de 1,5 à 3 nM.

*In vitro,* il suffit d'ajouter ladite homéoprotéine au milieu de culture des neurones. *In vivo,* elle peut être administrée par différentes voies, localement, notamment par injection ou infusion dans l'humeur vitrée, ou dans l'espace sous-orbital, ou sous forme de collyre, ou de pommade ophtalmique. Elle peut également être administrée à l'aide d'un dispositif de libération contrôlée, par exemple sous forme d'implant intra-oculaire. Le cas échéant, elle peut être administrée de manière systémique, par exemple par injection intraveineuse.

Les doses d'homéoprotéine à administrer *in vivo* pour obtenir la concentration souhaitée au contact des neurones ganglionnaires, peuvent aisément être déterminées et adaptées par l'homme de l'art en fonction notamment des modalités d'administration envisagées.

On peut également effectuer cette mise en contact en mettant les neurones ganglionnaires en présence de cellules transformées pour exprimer ou surexprimer, et sécréter ladite homéoprotéine. *In vitro,* ceci peut s'effectuer par co-culture de ces cellules transformées avec des neurones ganglionnaires. *In vivo,* on peut par exemple greffer dans la rétine des cellules transformées pour exprimer ou surexprimer, et sécréter ladite homéoprotéine.

On peut également, le cas échéant, associer ladite homéoprotéine avec un ou plusieurs autres principes actifs thérapeutiques, en administration conjointe ou séparée. Par exemple, dans le cadre du traitement du glaucome, on peut l'associer à une molécule ou une combinaison de molécules utilisée dans ce traitement, telles par exemple que celles décrites par WOODWARD & CHEN (2007, précité), et notamment une molécule ou une combinaison de molécules capables de diminuer la pression intra-oculaire.

La présente Invention sera mieux comprise à l'aide du complément de description qui va suivre, qui se réfère à des exemples démontrant l'activité d'une homéoprotéine Otx2 sur la survie des neurones ganglionnaires.

### EXEMPLE 1 : PRODUCTION ET PURIFICATION D'OTX2 RECOMBINANTE

La séquence codant pour l'isoforme-1 (SwissProt P32243) de l'homéoprotéine Otx2 humaine a été clonée, sous contrôle du promoteur *trc* inductible par l'isopropyl β-D-thiogalactoside (IPTG), dans le plasmide pTchTEV2 (dérivé d'un plasmide pTrcHis2 (Invitrogen), par remplacement du segment NcoI-HindIII par un linker permettant l'insertion d'un produit de PCR contenant la séquence codante d'Otx2 en amont et en phase avec le site de clivage pour la protéase rTEV, suivi de l'étiquette myc-his6 en position C-terminale, pour produire le plasmide pTrOtx2hTev.) La protéine recombinante exprimée par pTrOtx2hTev contient la séquence d'Otx2 fusionnée en C-terminal à une étiquette Myc et une étiquette 6xHis. Elle a été produite dans la souche d'*E*. *Coli* BL21 CodonPlus-RP (Novagen) plus RP » transformée par choc thermique. Après sélection des bactéries transformées sur boites de Pétri agar-LB-ampicilline à 37°C sur la nuit, l'expression de la protéine recombinante est induite par incubation une nuit à 37°C dans le milieu de culture auto-inducteur (IPTGlike) OverNight Express Instant TB Médium (Novagen). Après centrifugation, les bactéries sont reprises sur glace, dans un tampon de lyse (Tp : NaPO₄ 20mM, NaCl 0,5mM, avec inhibiteurs de protéases et sans EDTA), à 3 ml de Tp par gramme de culot bactérien) et lysées par 3 passages à la French Press à 1000 psi. Le lysat bactérien est centrifugé et le surnageant récupéré et filtré à 0,45 µm. Les protéines sont purifiées sur colonne Hitrap Chelating HP (Amersham) 1 ml chargée avec du NiSO₄ 0,1M, et le surnageant est passé à 0,5ml/min. Deux lavages sont effectués avec le Tp contenant de l'imidazole à 10mM, puis à 50mM. L'élution est faite par 10 fractions de 1 ml de Tp avec imidazole à 250mM. Une élution finale est faite avec le Tp et imidazole à 1M. La pureté des fractions d'élution a été contrôlée par électrophorèse SDS-Page, puis coloration au bleu de Coommassie.

Leur spécificité a été analysée par transfert de Western : deux µL de chaque fraction d'élution ont été mélangés avec du tampon de Laemmli et bouillis pendant 5 minutes. La séparation protéique a été effectuée par électrophorèse SDS-PAGE sur des gels d'acrylamide 12%. Les protéines sont électro-transférées sur membrane de nitro-cellulose. Après saturation dans un tampon contenant 5% de lait et 0.1% de Tween-20 dans du PBS 1X, la membrane est incubée avec l'anticorps primaire (anti-Otx2 polyclonal de rat au 1/200 ou anti-Myc monoclonal de souris au 1/1000) pendant la nuit à 4°C. Après rinçage, le filtre est incubé avec l'anticorps secondaire couplé à la peroxydase (HRP) pendant 1h. L'activité enzymatique de la peroxydase est révélée par chimioluminescence.

L'analyse en Western-Blot avec l'anticorps primaire anti-HPX, ou antiMyc permet de révéler une seule forte bande de migration au poids moléculaire attendu (environ 40kDa) pour la protéine HPX avec les étiquettes Myc et 6XHis.

Les 3 fractions les plus riches en Otx2 sont réunies : leur concentration en Otx2 est d'environ 200µg/ml. La préparation ainsi obtenue est dialysée contre un tampon Tris 50 mM/EDTA 0,5 mM/NaCl 200 mM, et stockées à -20°C dans ce même tampon contenant 45% de glycérol. Le glycérol est éliminé avant chaque expérience par dialyse contre le milieu de culture.

### EXEMPLE 2 : EFFET D'OTX2 SUR LA SURVIE DES NEURONES GANGLIONNAIRES RETINIENS AXOTOMISES.

### Cultures de cellules rétiniennes

L'effet de la protéine a été testé sur des neurones de rétine après dissociation et mise en culture. Deux protocoles ont été utilisés : d'une part des cultures mixtes comprenant tous les types de cellules rétiniennes, de l'autre des cultures de neurones ganglionnaires purifiés.

Toutes les expériences de cultures mixtes ont été réalisées sur des souris C57B16 adultes (de 6 à 10 semaines), et toutes les expériences de cultures purifiées de cellules ganglionnaires rétiniennes sur des rats adultes Long Evans de 8 semaines. Les souris et les rats ont été euthanasiés par dislocation cervicale. Les yeux ont été prélevés dans un délai de moins de 15 minutes, après désinfection périorbitaire au Mucocit (Biobloc), par dissection intra-orbitaire du globe oculaire. Les procédures utilisées sont en accord avec les recommandations de la CEE (86/609/EEC) et le Comité National Français pour l'utilisation des animaux du laboratoire.

### Cultures mixtes de cellules rétiniennes adultes dissociées

Le protocole utilisé est celui décrit par GAUDIN et al.(Invest Ophthalmol Vis Sci, 37, 2258-2268, 1996).

Des lamelles de verre stériles sont prétraitées avec de la Poly-D-Lysine (Sigma P-6407) à 2 µg/cm2 sur la nuit à 37°C puis de la laminine (Sigma L-2020) à 1 µg/cm2, 3 heures à 37°C. La dissection des rétines se fait dans le milieu CO2-indépendant et sans L-Glutamine (Gibco 18045-054). Les rétines sont morcelées aux ciseaux, rincées au PBS sans Ca2+, Mg2+ (Invitrogen 14190-185) avec glucose 0,6%, et EDTA à 0,5 mM, et incubées en présence de 0,2% papaïne (Worthington Biochemicals, 1 unité pour un tube contenant 10 rétines), pendant 15 minutes, à 37°C. La papaïne a été activée (30 minutes à 37°C) en ajoutant 1 unité de papaïne à 24 µL de solution activatrice de papaïne contenant 1,1 mM d'EDTA, 0,067 mM de β-mercaptoéthanol et 5,5 mM de L-Cystéine. L'hydrolyse est arrêtée par addition de 1 ml de milieu stop (Neurobasal A médium [Invitrogen 10888-022] et Sérum de Veau Foetal (SVF) 10% [Invitrogen 10270-098]), après addition de Dnase I (Sigma, 5 µg/ml). Les cellules sont dissociées, à la pipette pasteur rodée, dans le milieu stop, comptées avec le bleu de Trypan pour exclure les cellules mortes, ensemencées à différentes densités cellulaires, (de 75,000 à 400,000 cellules par puits) et maintenues en culture 6 jours. La protéine Otx2 recombinante est dialysée, prédiluée dans le milieu de culture et répartie dans les puits à différentes concentrations juste avant l'ensemencement cellulaire. Le milieu de culture, sans sérum, est composé de Neurobasal A médium (NBA) (Gibco 10888) supplémenté par : L-glutamine 5µM (Sigma G-6392), B27 complément 2,5µM (Gibco 17504-044), Glutamate-Aspartate 2,5µM (Gibco), AntibioticAntimycotic (Gibco 15240-096). Les cultures sont effectuées à 37°C dans des étuves à 95% d'air et 5% de CO2. Au 6ème jour de culture, les cellules sont fixées dans du paraformaldéhyde (PAF) 4% pendant 15 minutes puis rincées 3 fois avec du PBS et conservées à 4°C jusqu'à la réalisation de l'immunocytochimie.

Les RGCs survivant après 6 jours en culture mixte, sont identifiées par leur immunoréactivité pour deux marqueurs complémentaires: les anticorps anti-neurofilament 200 (NF-200 ; Sigma N-0142), et anti-neurofilament 68 (NF-68 ; Sigma N-5 139). Ils ont une spécificité respective de 91 et 88% (KONG and CHO, Life Sci, 64, 1773-1778, 1999).Leur sensibilité respective sur les grandes RGCs (taille >21 µm) est de 94 et 100%. Elle ont une sensibilité de 64 et 84% sur les petites RGCs (taille <14µm)(RUIZ-EDERRA et al., Mol Vis, 10, 83-92, 2004). Des critères morphologiques sont aussi utilisés : les RGCs ont une taille variable, et une forme ronde avec un noyau excentré.

Les procédures sont réalisées à température ambiante. Les cellules (fixées à J6) sont perméabilisées 5 minutes dans du PBS avec Triton X-100 0,2%, rincées dans du PBS 3 fois, saturées 30 minutes dans du PBS avec SVF 10% (tampon PBS-SVF), puis incubées avec le (ou les) anticorps primaire(s) dilués dans le même tampon pendant 2 heures Les cellules sont ensuite rincées 3 fois dans du PBS et incubées 1 heure avec le (ou les) anticorps secondaire(s)

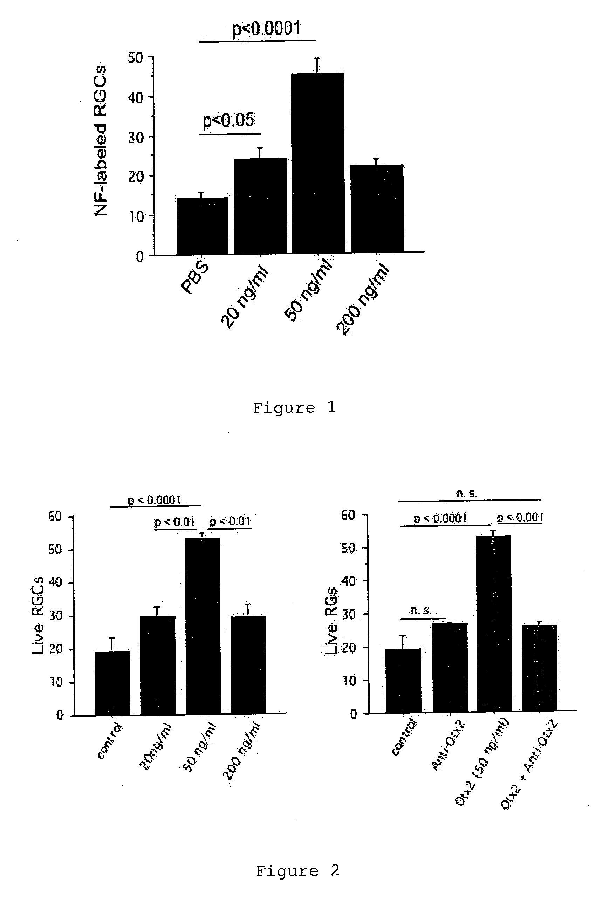

Les résultats obtenus sont illustrés par la Figure 1. On observe une survie maximale (x3) à 50ng/ml, soit 1,65nM. Un effet est visible dès 0,7nM.

### Cultures de cellules ganglionnaires rétiniennes adultes purifiées par immunopanning sur l'anticorps Thy-1

Le protocole utilisé est celui décrit par BARRES et al. (Neuron, 1, 791-803, 1988).

Le prétraitement des lamelles et le milieu de culture sans sérum sont les mêmes que pour les cultures mixtes. Sauf précision, les différentes incubations sont réalisées à température ambiante.

### Préparation de la suspension cellulaire

La dissection des rétines se fait dans du D-PBS (Invitrogen 14287-080). Les rétines sont rincées au D-PBS, puis incubées en présence de papaïne (Worthington Biochemicals, 165 unités pour un tube contenant 12 rétines), pendant 30 minutes à 37°C. La papaïne a été activée 5 minutes à 37°C en ajoutant 165 unités de papaïne à 5 ml de D-PBS et 1000 unités de DNase (Sigma D4527). L'hydrolyse est arrêtée par addition de 4 ml d'ovomucoïd 0,15%. Les cellules sont dissociées à la pipette Pasteur rodée, dans une solution d'ovomucoïd 0,15%, en présence de DNase et d'anticorps primaire de lapin anti-macrophage de rat (France Biochem AIA5 1240). La suspension cellulaire ainsi dissociée et préincubée est centrifugée, reprise dans 15 ml de D-PBS avec 0,02%BSA (Sigma A8806), puis filtrée sur filtre Nitex 48tm (Dutscher 074011).

### Préparation des boîtes de panning et anticorps utilisés

Pendant la nuit précédant la dissection des rétines, deux boîtes de Pétri dites « A » (150 mm, Dutscher 35-1058) sont incubées avec 20 ml de solution Tris-HCl 50mM pH 9,5 et 60 µL de l'anticorps secondaire chèvre anti-IgG de lapin (Interchim 111-005-003), et une boîte de Pétri dite « B » (100 mm, Dutscher 35-1029) avec 10 ml de solution Tris-HCl 50mM pH 9,5 et 30 µL de l'anticorps secondaire chèvre anti-IgM de souris (Interchim 1 15-005-020).Chaque boîte de panning est ensuite lavée 3 fois au PBS. Puis les boîtes A sont saturées avec du D-PBS 0,2%BSA. La boîte B est incubée 3 heures avec l'IgM de souris anti-Thy1 (T1 1D7, hybridome ECACC), puis lavée 4 fois au D-PBS.

### 1^{ère} étape de panning : soustraction des macrophages

La suspension cellulaire, pré-incubée avec l'anticorps primaire IgG de lapin anti-macrophage de rat, est incubée avec l'anticorps secondaire de chèvre anti-IgG de lapin sur la première boîte A pendant 36 minutes. Les cellules non adhérentes sont transférées sur la deuxième boîte A pour une deuxième incubation de 33 minutes.

### 2^{ème} étape de panning : sélection des CGRs

Les cellules non adhérentes sont filtrées sur filtre Nitex 48 tm et incubées pendant 45 minutes sur la boîte B contenant l'anticorps primaire IgM de souris anti -Thy1. La boîte B est ensuite lavée plusieurs fois (10 fois au moins) au D-PBS pour déloger progressivement les cellules non-adhérentes. Cette progression est surveillée sous microscope.

### Etape de décrochage à la trypsine des cellules adhérentes purifiées

La boîte B est rincée 2 fois avec Earle's Balanced Salt Solution (EBSS) (Sigma E6267) préchauffée à 37°C. Les cellules adhérentes sur la boîte B sont incubées avec une solution de trypsine contenant 4 ml d'EBSS et 200 µL de trypsine 2,5% (Sigma T9201), 10 minutes à 37°C. La trypsine est inactivée par une solution de 4 ml de D-PBS-Fetal Bovine Sérum (FBS) 30%. Les cellules sont détachées en pipetant doucement avec la solution de blocage de la trypsine, puis centrifugées, et comptées (exclusion des cellules mortes avec le bleu de Trypan).

Otx2 est dialysée, prédiluée dans le milieu de culture puis déposée dans les puits avant l'ensemencement des cellules à une densité de 20,000 cellules par puits. Dans certaines expériences, Otx2 a été pré-incubée avec un anticorps polyclonal anti-OTx2 (Neuromics) au 1/1000, 30 minutes à 37°C.

Un test de survie des cellules est réalisé à J1 pour évaluer le nombre moyen de RGCs vivantes ensemencées initialement par puits (sur 4 lamelles), puis à J6 pour évaluer la proportion de celles ayant survécu en culture dans les différentes conditions (3 à 6 lamelles par condition). Les RGCs purifiées sont incubées pendant 2 heures à 37°C avec un mélange de deux réactifs : l'AM calcéine et l'éthidium (Live Dead Viability Cytotoxicity Kit, Invitrogen, L3224). L'AM calcéine ne fluoresce (en vert) que si elle pénètre dans une cellule vivante où elle est hydrolysée en calcéine fluorescente. L'éthidium ne pénètre que dans les cellules mortes aux membranes endommagées et ne fluoresce en rouge qu'en interagissant avec leur ADN. Les deux marqueurs ne fluorescent que s'ils pénètrent dans les cellules, il n'y a donc pas de bruit de fond. L'analyse est faite directement au microscope en transposant les lamelles (8mm) sur des porte-lamelles spéciaux à cet effet, dans 75µL du milieu de culture incubé à 37°C avec les réactifs du test Live-Dead.

La Figure 2 illustre les résultats obtenus. Ces résultats montrent que la même concentration de 1.65nM est optimale pour la survie (x3) et que l'effet d'Otx2 est annulé après préincubation de la protéine avec l'anticorps neutralisant anti-Otx2, qui n'a pas d'effet par lui-même. L'effet de survie est donc bien dû à Otx2 et non à un possible contaminant.

### EXEMPLE 3 : COMPARAISON DES EFFETS D'OTX2, DU MILIEU CONDITIONNE DE CULTURES MIXTES DE RETINES, ET DU BDNF SUR LA SURVIE DES NEURONES GANGLIONNAIRES RETINIENS.

Les effets d'Otx2 sur la survie des neurones ganglionnaires ont été comparés avec ceux de facteurs de survie des neurones ganglionnaires adultes précédemment décrits dans la littérature : le milieu conditionné de cultures mixtes de rétines (FUCHS et al., Invest Ophthalmol Vis Sci, 46, 2983-2991, 2005), et le BDNF (Brain Derived Neurotrophic Factor (JOHNSON et al., J Neurosci, 6, 3031-3038, 1986).

Les expérimentations ont été effectuées sur des cultures de cellules ganglionnaires adultes purifiées par immunopanning, comme décrit à l'Exemple 2 ci-dessus.

Otx2 et le BNDF ont été utilisés à la concentration de 50ng/ml dans le milieu de culture.

Le milieu conditionné de cultures mixtes de rétines est préparé à partir de cultures mixtes réalisées selon le protocole décrit à l'exemple 2 ci-dessus. Le milieu de culture initial contient 10% de SVF pour permettre la prolifération des cellules gliales de Müller. Les cellules sont cultivées dans ces conditions jusqu'à confluence (environ 10 jours). Après 4 lavages au NBA, le milieu de culture est changé pour un milieu de culture chimiquement défini, sans sérum (NBA+ B27 2%) pour 2 jours supplémentaires. Ce milieu conditionné (MC) est alors récupéré, centrifugé, aliquoté puis congelé dans l'azote liquide.

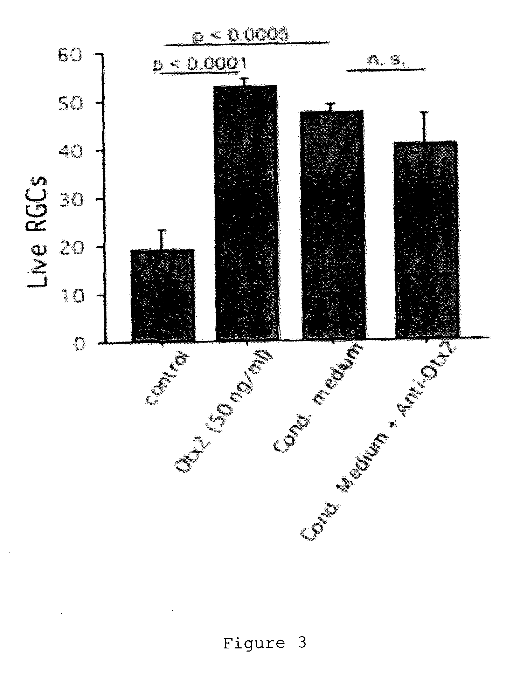

Les résultats sont illustrés par la Figure 3. Ces résultats montrent qu'Otx2 à 50ng/ml (1,65nM) est aussi efficace, sinon plus que le milieu conditionné. La préincubation du milieu conditionné avec l'anticorps anti-Otx2 ne modifie pas son effet, ce qui montre que cet effet n'est pas dû à son contenu en Otx2. Le BDNF (Brain Derived Neurotrophic Factor) à 50ng/ml donne une activité similaire à celle du milieu conditionné (résultats non-illustrés).

Il ressort des expériences décrites ci-dessus qu'Otx2 est un nouveau facteur de survie pour les neurones ganglionnaires adultes, et que son activité est égale ou supérieure à celle du BDNF ou du milieu conditionné.

### EXEMPLE 4 : EFFETS D'OTX2 SUR LA SURVIE IN VIVO DES NEURONES GANGLIONNAIRES RETINIENS.

L'effet d'Otx2 sur la survie des neurones ganglionnaires rétiniens a été confirmé *in vivo* dans un modèle murin.

Le modèle choisi est l'intoxication au N-methyl-D-aspartate (NMDA). La survie des neurones ganglionnaires a été déterminée en mesurant le niveau d'expression de Brain 3A (Brn3A), un facteur de transcription qui dans la rétine, est spécifiquement exprimé dans les neurones ganglionnaires RGCs (XIANG et al., J. Neurosci., 15, 4762-4785, 1995).

Des souris C57 B16 ont reçu dans l'oeil droit, 1 µl de tampon d'injection (PBS ou NaCl 9 %) contenant soit 30ng d'Otx2, soit 1mM de NMDA, soit 1mM de NMDA additionné de 3 ng ou de 30 ng d'Otx2, et dans l'oeil gauche, le même volume de tampon d'injection, sans additif.

Au bout de 4 jours, les animaux sont sacrifiés, les rétines prélevées, et l'ARNm en est extrait.

Le niveau d'expression de l'ARNm de Brn3A a été déterminé par RT-PCR quantitative, en utilisant le gène de l'hypoxanthine phosphoribosyltransférase (HPRT) comme gène de référence, et le rapport entre l'expression de l'ARNm de Brn3A dans l'oeil droit et dans l'oeil gauche a été calculé.

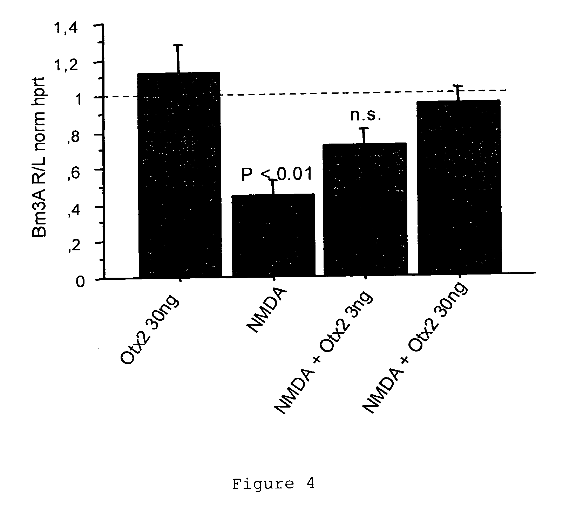

Les résultats sont illustrés par la Figure 4. En abscisse, sont indiqués les additifs utilisés ; en ordonnée, est indiqué le rapport entre les quantités d'ARNm Brn3A (normalisées par rapport à l'ARNm HPRT) dans l'oeil droit et dans l' oeil gauche.

Ces résultats montrent qu'Otx2 seul n'a pas d'effet significatif sur le niveau d'expression de Brn3A (et donc sur la quantité de neurones ganglionnaires) dans la rétine. Le NMDA, administré seul, diminue significativement (d'environ 60%) la quantité des neurons ganglionnaires, et l'addition de 3ng d'Otx2 ne diminue pas significativement les effets toxiques du de NMDA. En revanche l'addition de 30 ng d'Otx2 protège totalement les neurones ganglionnaires contre les effets toxiques du NMDA.

## Revendications

1. Homéoprotéine dont l'homéodomaine possède au moins 98% d'identité de séquence avec les résidus 38-97 de la séquence SEQ ID NO: 1 et contient un résidu lysine en position 50, et dont la séquence polypeptidique globale possède au moins 90% d'identité avec la séquence SEQ ID NO: 1 ou avec la séquence SEQ ID NO: 2 pour l'utilisation comme médicament pour la prévention ou le traitement de la dégénérescence des neurones ganglionnaires rétiniens.

2. Homéoprotéine pour l'utilisation selon la revendication 1, **caractérisée en ce que** ladite dégénérescence des neurones ganglionnaires rétiniens intervient au cours du glaucome.

3. Utilisation d'une homéoprotéine dont l'homéodomaine possède au moins 98% d'identité de séquence avec les résidus 38-97 de la séquence SEQ ID NO: 1 et contient un résidu lysine en position 50, et dont la séquence polypeptidique globale possède au moins 90% d'identité avec la séquence SEQ ID NO: 1 ou avec la séquence SEQ ID NO: 2, ou d'une composition comprenant ladite homéoprotéine pour augmenter la survie de neurones ganglionnaires rétiniens en culture.

## Patentansprüche

1. Homeoprotein, dessen Homeodomäne wenigstens 98% Sequenzidentität mit den Resten 38-97 der Sequenz SEQ ID NO:1 besitzt und einen Lysinrest an Position 50 enthält und dessen Gesamtpolypeptidsequenz wenigstens 90% Identität mit der Sequenz SEQ ID NO:1 oder mit der SEQ ID NO:2 besitzt, zur Verwendung als Medikament für die Prävention oder die Behandlung der Degeneration der Retina-Ganglionneuronen.

2. Homeoprotein zur Verwendung gemäß Anspruch 1, **dadurch gekennzeichnet, dass** die Degeneration der Retina-Ganglionneuronen bei Glaukom auftritt.

3. Verwendung eines Homeoproteins, dessen Homeodomäne wenigstens 98% Sequenzidentität mit den Resten 38-97 der Sequenz SEQ ID NO:1 besitzt und einen Lysinrest in Position 50 enthält, und dessen Gesamtpolypeptidsequenz wenigstens 90% Identität mit der Sequenz SEQ ID NO:1 oder mit der Sequenz SEQ ID NO:2 besitzt, oder einer Zusammensetzung, die das Homeoprotein umfasst, um das überlegen von Retina-Ganglionneuronen in Kultur zu erhöhen.

## Claims

1. Homeoprotein whose homeodomain has at least 98% sequence identity with residues 38-97 of the sequence SEQ ID NO: 1 and contains a lysine residue at position 50 of said homeodomain and whose overall polypeptide sequence has at least 90% identity with the sequence SEQ ID NO: 1 or the sequence SEQ ID NO: 2, for use as a medicament for the prevention or treatment of retinal ganglion neuron degeneration.

2. Homeoprotein for the use according to claim 1, **characterized in that** said retinal ganglion neuron degeneration occurs in glaucoma.

3. Use of a homeoprotein whose homeodomain has at least 98% sequence identity with residues 38-97 of the sequence SEQ ID NO: 1 and contains a lysine residue at position 50 of said homeodomain and whose overall polypeptide sequence has at least 90% identity with the sequence SEQ ID NO: 1 or the sequence SEQ ID NO: 2, or a composition comprising said Homeoprotein, for increasing the survival of retinal ganglion neurons in culture.
